# FASCICULE DE BREVET EUROPEEN

(11) **EP 2 117 510 B1**
(45) Date de publication et mention de la délivrance du brevet: **09.03.2011**
(21) Numéro de dépôt: 08761739.5
(22) Date de dépôt: 04.01.2008
(51) Int. Cl.: A61K 9/107, A61K 38/13

(54) **COMPOSITION PHARMACEUTIQUE PARENTÉRALE ET SON PROCEDE DE PREPARATION.**
PARENTERALE PHARMAZEUTISCHE ZUSAMMENSETZUNG UND VERFAHREN ZU DEREN HERSTELLUNG
PARENTERAL PHARMACEUTICAL COMPOSITION AND METHOD FOR PREPARING THE SAME

(30) Priorité: 09.01.2007 FR 0700127
(43) Date de publication de la demande: 18.11.2009
(73) Titulaire: Physica Pharma, 33600 Pessac (FR)
(72) Inventeur: BROUSSAUD, Olivier, F-33000 Bordeaux (FR); POUGNAS, Jean-Luc, F-33500 Libourne (FR); CALVET, Nicolas, F-33200 Bordeaux (FR)
(74) Mandataire: Noel, Chantal Odile
(86) Numéro de dépôt international: PCT/FR2008/000015
(87) Numéro de publication internationale: WO 2008/099084

(56) Documents cités:
- WO-A-97/25977
- WO-A-2007/006334
- JEON IL-SOON ET AL: "Preparation and evaluation of paclitaxel nano-particle delivery system for parenteral formulations" BIOSIS, août 2005 (2005-08), XP002429519
- GONZALEZ ET AL: "In vitro characterization of an emulsion pre-concentrate formulation designed for the oral administration of poorly soluble compounds" PROCEEDINGS OF THE INTERNATIONAL SYMPOSIUM ON CONTROLLED RELEASE BIOACTIVE MATERIALS, XX, XX, vol. 28, 23 juin 2001 (2001-06-23), pages 754-755, XP009089496 ISSN: 1022-0178
- JUMAA MUHANNAD ET AL: "Lipid emulsions as a novel system to reduce the hemolytic activity of lytic agents: Mechanism of the protective effect" EUROPEAN JOURNAL OF PHARMACEUTICAL SCIENCES, vol. 9, no. 3, janvier 2000 (2000-01), pages 285-290, XP002451267 ISSN: 0928-0987
- WPI WORLD PATENT INFORMATION DERWENT, DERWENT, GB, vol. 43, no. 92, 9 septembre 1992 (1992-09-09), XP002031188

## Description

La présente invention concerne une composition pharmaceutique sous forme d'émulsion aqueuse qui comprend un principe actif peu soluble dans l'eau, à l'exclusion du Propofol, et qui est notamment apte à être administrée par injection intraveineuse, et un procédé pour la préparation de cette composition. L'invention s'applique de manière plus générale à des administrations parentérales de cette composition, par exemple par application cutanée.

Le Propofol (i.e. le 2,6-diisopropylphénol) est un agent anesthésiant liquide bien connu que l'on utilise depuis le début des années 1980 dans les compositions pharmaceutiques sous forme d'émulsions aqueuses pour injections intraveineuses. A cause de la faible solubilité du Propofol dans l'eau qui résulte de sa nature lipophile, on a cherché à optimiser les formulations intraveineuses contenant du Propofol afin de dissoudre complètement ce principe actif dans une composition induisant une tolérance satisfaisante de l'organisme.

La Demande de Brevet PCT/EP2005/008739 du 8 juillet 2005 au nom de la Demanderesse a eu notamment pour objet de remédier aux inconvénients suivants des compositions antérieures sous forme d'émulsions aqueuses injectables à base de Propofol qui étaient généralement, soit de type macroémulsion opaque à cause d'une quantité élevée de triglycérides et/ou de phospholipides, soit de type microémusion claire dépourvue de triglycérides mais présentant un rapport massique (m/m) système solubilisant / Propofol relativement élevé pouvant entraîner des effets secondaires indésirables après injection intraveineuse. Selon cette Demande de Brevet, le système solubilisant comprenait, à titre de tensioactif ionique, un sel métallique monovalent d'un acide gras ayant de 5 à 23 atomes de carbone et, à titre de tensioactif non ionique, un polyéthylène glycol hydroxystéarate.

Il est fait référence aux documents suivants:
D1 JEON IL-SOON ET AL: "Preparation and evaluation of paclitaxel nano-particle delivery system for parenteral formulations" BIOSIS, août 2005 (2005-08), XP002429519
D2 GONZALEZ ET AL: "In vitro characterization of an emulsion pre-concentrate formulation D2 designed for the oral administration of poorly soluble compounds" PROCEEDINGS OF THE INTERNATIONAL SYMPOSIUM ON CONTROLLED RELEASE BIOACTIVE MATERIALS, XX, XX, vol. 28, 23 juin 2001 (2001-06-23), pages 754-755, XP009089496 ISSN: 1022-0178
D3 JUMAA MUHANNAD ET AL: "Lipid emulsions as a novel system to reduce the hemolytic D3 activity of lytic agents: Mechanism of the protective effect" EUROPEAN JOURNAL OF PHARMACEUTICAL SCIENCES, vol. 9, no. 3, janvier 2000 (2000-01), pages 285-290, XP002451267 ISSN: 0928-0987
D4 WO 97/25977 A (CIBA GEIGY AG [CH]; TIEMESSEN HARRY [DE]) 24 juillet 1997 (1997-07-24)
D5 WPI WORLD PATENT INFORMATION DERWENT, DERWENT, GB, vol. 43, no. 92, 9 septembre 1992 (1992-09-09), XP002031188

D1, D2, D4, D5 ne décrivent pas le système solubilisant comprenant au moins un sel métallique monovalent d'un acide gras ayant 5 à 23 atomes de carbones tel que l'oléate de sodium, et au moins un PEG hydrostéarate (solutol H15).

D3 ne concerne pas une composition pharmaceutique per se, aucun principe actif n'étant testé, ni même mentionné dans cet article. Il ne vise pas à résoudre le problème de solubilisation d'un principe actif sensiblement insoluble dans l'eau.

On connaît par ailleurs d'autres compositions pharmaceutiques injectables sous forme d'émulsions aqueuses, dont le principe actif présente toujours une solubilité faible dans l'eau mais n'est pas constitué de Propofol. Parmi ces compositions connues, on peut par exemple citer celles décrites dans le document EP-A-874 621, qui comprennent :
- à titre de principe actif, une cyclosporine particulière qui est la [3'-desoxy-3-oxo-MeBmt]¹ -[Val]² -cyclosporine, et
- à titre de système solubilisant de ce principe actif, soit un phospholipide tel que le palmitoyl oléoyl phosphatidylglycérol de sodium (POPG-Na), soit un sel d'un acide carboxylique saturé ou insaturé ayant de 12 à 24 atomes de carbone tel que l'oléate de sodium.

Un inconvénient majeur de la composition selon ce dernier document réside notamment dans la taille moyenne comprise entre 250 nm et 500 nm des micelles obtenues dans les émulsions formées, ce qui peut potentiellement entraîner des problèmes de stérilisation du produit fini lorsque celle-ci ne peut se faire par la chaleur (en autoclave) ou par radio-stérilisation. Dans ce cas, il est nécessaire de recourir à une filtration stérilisante sur membrane dont la taille des pores est de l'ordre de 220 nm. Le produit fini selon ce document doit alors présenter une taille moyenne des micelles inférieure à 220 nm, afin d'éviter tout risque de rétention du principe actif sur la membrane ou de déstabilisation du système dispersé.

Un but de la présente invention est de remédier à cet inconvénient, et ce but est atteint en ce que la Demanderesse vient de découvrir d'une manière surprenante qu'un système solubilisant comprenant en combinaison au moins un sel métallique monovalent pharmaceutiquement acceptable d'un acide gras ayant de 5 à 23 atomes de carbone et au moins un polyéthylène glycol hydroxystéarate, permet d'optimiser la solubilisation d'un principe actif peu soluble dans l'eau, à l'exclusion du Propofol et pouvant être avantageusement un polypeptide cyclique sous forme de poudre tel que la cyclosporine A, pour l'obtention d'une composition pharmaceutique sous formé d'émulsion aqueuse apte à être administrée par injection intraveineuse et présentant des micelles de taille moyenne inférieure à 200 nm, permettant en cas de besoin une stérilisation par filtration sur une membrane.

On notera que la présente invention réside dans l'effet de synergie inattendu et avantageux de la combinaison de ces deux tensioactifs ionique et non ionique, en comparaison de l'état de l'art antérieur qui divulgue isolément, soit ce sel métallique avec un principe actif selon l'invention (voir le document précité EP-A-874 621), soit le polyéthylène glycol hydroxystéarate avec le Propofol (voir par exemple le document WO-A-00/78301).

Selon une autre caractéristique avantageuse de l'invention, ladite composition comprend de l'eau selon une fraction massique égale ou supérieure à 30 % et, de préférence, égale ou supérieure à 50 %, cette composition pouvant être ainsi par exemple directement injectée dans les vaisseaux sanguins (i.e. avec ou sans dilution de cette composition antérieure à l'injection).

Avantageusement, les micelles présentes dans la composition selon l'invention ont une taille moyenne inférieure à 100 nm et, encore plus avantageusement, inférieure ou égale à 60 nm. Il en résulte que ladite composition peut être alors translucide, voire sensiblement claire, alors qu'elle est relativement opaque pour des tailles moyennes de micelles comprises entre 100 nm et 200 nm.

De préférence, le principe actif utilisable selon l'invention est à base d'une poudre solide à la température ambiante, à la différence du Propofol qui se présente sous la forme d'un liquide à température ambiante.

Egalement à titre préférentiel, ce principe actif selon l'invention présente une solubilité aqueuse à la température ambiante (environ 22° C) qui est inférieure à 300 µg/mL et, de préférence, inférieure à 150 µg/mL (la solubilité du Propofol dans l'eau à cette même température est de l'ordre de 160 µg/mL).

A titre plus préférentiel, le principe actif de l'invention présente une solubilité aqueuse à la température ambiante qui est inférieure à 125 µg/mL et, .à titre encore plus préférentiel, inférieure à 100 µg/mL voire inférieure ou égale à 20 µg/mL, comme cela est le cas notamment de la cyclosporine A (polypeptide monocyclique à 11 acides aminés) dont la solubilité dans l'eau à la température ambiante est d'environ 6 µg/mL.

D'une manière générale, on notera que le principe actif utilisable selon l'invention peut être utilisé indifféremment sous sa forme base ou sous une forme de sel, d'hydrate, de solvate ou d'isomère.

Selon une autre caractéristique de l'invention, ladite composition pharmaceutique est avantageusement dépourvue de tout triglycéride et de tout phospholipide.

De préférence, l'acide gras dudit ou de l'un au moins desdits sel(s) métallique(s) est un monoacide carboxylique ayant de 8 à 18 atomes de carbone et, à titre encore plus préférentiel, ledit acide gras est un acide aliphatique saturé ou insaturé et il comporte de 16 à 18 atomes de carbone, tel que l'acide oléique.

Quant au métal utilisé pour ledit ou l'un au moins desdits sel(s) métallique(s), il s'agit avantageusement du sodium ou du potassium.

Encore plus préférentiellement, ledit ou l'un au moins desdits sel(s) métallique(s) est l'oléate de sodium.

Egalement à titre préférentiel, ledit ou l'un au moins desdits polyéthylène glycol hydroxystéarate(s) comporte de 10 à 25 unités oxyde d'éthylène et, à titre encore plus préférentiel, il comporte 15 unités oxyde d'éthylène (il s'agit alors du PEG-15 hydroxystéarate).

Ainsi, selon un exemple préférentiel de réalisation de l'invention, ledit système solubilisant comprend en combinaison de l'oléate de sodium et du PEG-15 hydroxystéarate (par exemple commercialisé sous la dénomination « Solutol HS 15 »), avantageusement mélangés à de la cyclosporine A.

Avantageusement, ladite composition pharmaceutique selon l'invention comprend en outre un système adjuvant incluant par exemple :
- au moins un solvant, tel qu'un polyéthylène glycol, de l'éthanol ou du propylène glycol, à titre d'adjuvant(s) et/ou
- au moins un composé polyoxyéthylénique pouvant être par exemple un poloxamer (e.g. le poloxamer 188) et/ou un ester polyoxyéthylénique de sorbitanne (e.g. le mono-oléate polyoxyéthylénique de sorbitanne à 20 unités oxyéthylène, encore appelé « polysorbate 80 »), également à titre d'adjuvant(s), et/ou
- un agent de contrôle du pH, un agent de contrôle de l'osmolarité et/ou un agent de structure (permettant de stabiliser le système dispersé), également à titre d'adjuvant(s).

Avantageusement, ladite composition pharmaceutique selon l'invention est telle que le rapport massique (m/m) système solubilisant / principe actif est inférieur ou égal à 15 et, encore plus avantageusement, inférieur ou égal à 10 voire inférieur ou égal à 5.

Selon une autre caractéristique de l'invention, le rapport massique (m/m) dudit ou desdits sel(s) métallique(s) sur ledit principe actif est avantageusement inférieur à 1 et, de préférence, inférieur à 0,05.

Egalement avantageusement, le rapport massique (m/m) dudit ou desdits polyéthylène glycol hydroxystéarate(s) sur le(s)dit(s) sel(s) métallique(s) appartient à un domaine allant de 10 à 130.

Selon l'invention, ladite composition pharmaceutique peut être préparée en ajoutant un principe actif sensiblement insoluble dans l'eau, à l'exclusion du Propofol, ce principe actif étant préalablement dissous dans un solvant, à un mélange aqueux comprenant :
(i) de l'eau, de préférence selon une fraction massique dans la composition égale ou supérieure à 30 % voire égale ou supérieure à 50 %,
(ii) un système solubilisant dudit principe actif qui comprend au moins un sel métallique monovalent pharmaceutiquement acceptable d'un acide gras ayant de 5 à 23 atomes de carbone, tel que l'oléate de sodium, et au moins un polyéthylène glycol hydroxystéarate, tel que le PEG-15 hydroxystéarate, et
(iii) un système adjuvant comprenant au moins :
   - au moins un solvant (identique ou différent audit solvant de dissolution du principe actif), tel qu'un polyéthylène glycol, de l'éthanol ou du propylène glycol, et/ou
   - au moins un composé polyoxyéthylénique, tel qu'un poloxamer (e.g. le poloxamer 188) et/ou un ester polyoxyéthylénique de sorbitanne (e.g. le mono-oléate polyoxyéthylénique de sorbitanne à 20 unités oxyéthylène, encore appelé « polysorbate 80 »), et/ou
   - un agent de contrôle du pH, un agent de contrôle de l'osmolarité et/ou un agent de structure (permettant de stabiliser le système dispersé).

Selon un exemple de mise en oeuvre de ce procédé de préparation selon l'invention, dans une première étape, on dissout dans au moins un solvant ledit principe actif, utilisé sous une forme physique solide à la température ambiante.

Après cette dissolution, on ajoute le tensioactif non ionique, tel que le PEG-15 hydroxystéarate. Après homogénéisation, on ajoute ledit système adjuvant, tels que les agents de structure et d'osmolarité. Parallèlement à cette seconde étape, on prépare la phase aqueuse en dissolvant dans de l'eau le tensioactif ionique, tel que l'oléate de sodium.

On ajoute alors cette phase aqueuse à la solution contenant le principe actif et ledit tensioactif non ionique, jusqu'à l'obtention d'une solution homogène. Puis, si nécessaire, on y introduit sous agitation des agents de pH.

Les caractéristiques précitées de la présente invention, ainsi que d'autres, seront mieux comprises à la lecture de la description suivante d'un exemple de réalisation de l'invention, donné à titre illustratif et non limitatif.

### Exemple de préparation d'une composition pharmaceutiques selon l'invention à base de cyclosporine A :

On a utilisé pour préparer cette composition des moyens de mélangeage couramment utilisés en laboratoire, et l'on a mis en oeuvre le procédé suivant :
- étape (i) : dans un récipient approprié, on a mélangé la cyclosporine avec un solvant, tel qu'un polyéthylène glycol, puis on a chauffé ce mélange à 40° C ;
- étape (ii) : une fois la cyclosporine dissoute, on a ajouté à la solution obtenue un tensioactif non ionique, tel que le PEG-15 hydroxystéarate. On a alors chauffé l'ensemble à 40° C sous agitation jusqu'à complète homogénéisation. Une fois homogène, on a refroidi le produit obtenu jusqu'à la température ambiante ;
- étape (iii) : pour l'obtention de la phase aqueuse, on a dissous dans de l'eau un tensioactif ionique, tel que de l'oléate de sodium, jusqu'à l'obtention d'une solution translucide ; puis
- étape (iv) : on a ajouté sous agitation cette phase aqueuse à la solution obtenue à l'étape (ii), jusqu'à l'obtention d'une solution homogène claire ou translucide.

On a mesuré la taille moyenne des micelles obtenues dans l'émulsion aqueuse correspondante grâce à un appareil commercialisé par la société MALVERN sous la dénomination « ZetaSizer Nano ».

La composition selon l'invention ainsi préparée présentait la formulation suivante, exprimée en fractions massiques (m/m) d'ingrédients :

| | |
|---|---|
| Principe actif | |
| Cyclosporine A | 0,5 % |
| Système solubilisant | |
| Oléate de sodium | 0,05 % |
| PEG-15 hydroxystéarate | 5 % |
| (Solutol HS 15) | |
| Système adjuvant | |
| PEG 400 | 2 % |
| Eau à injecter | jusqu'à 100 %, i.e. 92,45 %. |

Cette composition selon l'invention présentait une taille moyenne de micelles d'environ 60 nm, lui conférant ainsi un aspect clair.

On notera que le rapport massique (m/m) système solubilisant / principe actif de cette composition était sensiblement égal à 10 ce qui, combiné à la taille moyenne de micelles relativement réduite et à la fraction massique d'eau très élevée (plus de 90 %), rendait la composition obtenue injectable telle que ou après dilution dans les vaisseaux sanguins avec des risques minimisés d'altération des propriétés physiologiques de l'organisme (e.g. intolérance, hémolyse, etc.).

## Revendications

1. Composition pharmaceutique sous forme d'émulsion aqueuse comprenant un principe actif sensiblement insoluble dans l'eau, à l'exclusion du Propofol, et un système solubilisant de ce principe actif, ladite composition étant apte à être administrée par injection intraveineuse, **caractérisée en ce que** ledit système solubilisant comprend au moins un sel métallique monovalent pharmaceutiquement acceptable d'un acide gras ayant de 5 à 23 atomes de carbone, et au moins un polyéthylène glycol hydroxystéarate.

2. Composition pharmaceutique selon la revendication 1, **caractérisée en ce qu'**elle comprend de l'eau selon une fraction massique égale ou supérieure à 30 %, de préférence égale ou supérieure à 50 %, ladite composition pouvant être par exemple directement injectée dans les vaisseaux sanguins.

3. Composition pharmaceutique selon une des revendications précédentes, **caractérisée en ce qu'**elle comprend des micelles présentant une taille moyenne inférieure à 200 nm, de préférence inférieure à 100 nm et, plus préférablement, inférieure ou égale à 60 nm.

4. Composition pharmaceutique selon une des revendications précédentes, **caractérisée en ce que** ledit principe actif est à base d'une poudre solide à la température ambiante.

5. Composition pharmaceutique selon la revendication 4, **caractérisée en ce que** ledit principe actif présente une solubilité aqueuse à la température ambiante qui est inférieure à 300 µg/mL, de préférence inférieure à 150 µg/mL, plus préférablement inférieure à 125 µg/mL et, le plus préférablement, inférieure à 100 µg/mL.

6. Composition pharmaceutique selon la revendication 5, **caractérisée en ce que** ladite solubilité aqueuse est inférieure ou égale à 20 µg/mL.

7. Composition pharmaceutique selon une des revendications précédentes, **caractérisée en ce qu'**elle est dépourvue de tout triglycéride et de tout phospholipide.

8. Composition pharmaceutique selon une des revendications précédentes, **caractérisée en ce que** l'acide gras dudit ou de l'un au moins desdits sel(s) métallique(s) est un monoacide carboxylique ayant de 8 à 18 atomes de carbone.

9. Composition pharmaceutique selon la revendication 8, **caractérisée en ce que** ledit acide gras est un acide aliphatique saturé ou insaturé et comporte de 16 à 18 atomes de carbone, tel que l'acide oléique.

10. Composition pharmaceutique selon une des revendications précédentes, **caractérisée en ce que** le métal dudit ou de l'un au moins desdits sel(s) métallique(s) est le sodium ou le potassium.

11. Composition pharmaceutique selon les revendications 9 et 10, **caractérisée en ce que** ledit ou l'un au moins desdits sel(s) métallique(s) est l'oléate de sodium.

12. Composition pharmaceutique selon une des revendications précédentes, **caractérisée en ce que** ledit ou l'un au moins desdits polyéthylène glycol hydroxystéarate(s) comporte de 10 à 25 unités oxyde d'éthylène, de préférence 15 unités oxyde d'éthylène.

13. Composition pharmaceutique selon une des revendications précédentes, **caractérisée en ce qu'**elle comprend en outre au moins un composé polyoxyéthylénique à titre d'adjuvant(s), de préférence un poloxamer ou bien un ester polyoxyéthylénique de sorbitanne, tel que le mono-oléate polyoxyéthylénique de sorbitanne à 20 unités oxyéthyléniques (polysorbate 80).

14. Composition pharmaceutique selon une des revendications précédentes, **caractérisée en ce qu'**elle comprend en outre au moins un solvant, tel qu'un polyéthylène glycol, de l'éthanol ou un propylène glycol, à titre d'adjuvant(s).

15. Composition pharmaceutique selon les revendications 4 et 6, **caractérisé en ce que** ledit principe actif est un polypeptide cyclique, tel que la cyclosporine A.

16. Composition pharmaceutique selon une des revendications précédentes, **caractérisée en ce que** le rapport massique (m/m) système solubilisant / principe actif est inférieur ou égal à 15.

17. Composition pharmaceutique selon la revendication 16, **caractérisée en ce que** le rapport massique (m/m) système solubilisant / principe actif est inférieur ou égal à 10 et, de préférence, inférieur ou égal à 5.

18. Composition pharmaceutique selon une des revendications précédentes, **caractérisée en ce que** le rapport massique (m/m) dudit ou desdits sel(s) métallique(s) sur ledit principe actif est inférieur à 1 et, de préférence, inférieur à 0,05.

19. Composition pharmaceutique selon une des revendications précédentes, **caractérisée en ce que** le rapport massique (m/m) dudit ou desdits polyéthylène glycol hydroxystéarate(s) sur le(s)dit(s) sel(s) métallique(s) appartient à un domaine allant de 10 à 130.

20. Procédé de préparation d'une composition pharmaceutique selon une des revendications précédentes, **caractérisé en ce qu'**il comprend l'ajout d'un principe actif sensiblement insoluble dans l'eau, à l'exclusion du Propofol, ce principe actif étant préalablement dissous dans un solvant, à un mélange aqueux comprenant :
(i) de l'eau, de préférence selon une fraction massique dans ladite composition égale ou supérieure à 30 %,
(ii) un système solubilisant dudit principe actif qui comprend au moins un sel métallique monovalent pharmaceutiquement acceptable d'un acide gras ayant de 5 à 23 atomes de carbone, tel que l'oléate de sodium, et au moins un polyéthylène glycol hydroxystéarate, tel que le PEG-15 hydroxystéarate, et
(iii) un système adjuvant comprenant au moins :
- un solvant identique ou différent audit solvant de dissolution du principe actif, tel qu'un polyéthylène glycol, de l'éthanol ou du propylène glycol, et/ou
- un composé polyoxyéthylénique, tel qu'un poloxamer (e.g. le poloxamer 188) et/ou un ester polyoxyéthylénique de sorbitanne (e.g. le mono-oléate polyoxyéthylénique de sorbitanne à 20 unités oxyéthylène, encore appelé « polysorbate 80 »), et/ou
- un agent de contrôle du pH, un agent de contrôle de l'osmolarité et/ou un agent de structure.

## Claims

1. Pharmaceutical composition in the form of an aqueous emulsion comprising an active ingredient that is substantially insoluble in water, excluding Propofol, and a solubilising system for this active ingredient, the said composition being suitable for administration by intravenous injection, **characterised in that** the said solubilising system comprises at least one pharmaceutically acceptable monovalent metal salt of a fatty acid having 5 to 23 carbon atoms, and at least one polyethylene glycol hydroxystearate.

2. Pharmaceutical composition according to claim 1, **characterised in that** it comprises water in a fraction by mass greater than or equal to 30%, preferably greater than or equal to 50%, the said composition being capable of being injected directly into the blood vessels, for example.

3. Pharmaceutical composition according to one of the preceding claims, **characterised in that** it comprises micelles having an average size of less than 200 nm, preferably less than 100 nm, and more preferably less than or equal to 60 nm.

4. Pharmaceutical composition according to one of the preceding claims, **characterised in that** the active ingredient is based on a powder that is solid at ambient temperature.

5. Pharmaceutical composition according to claim 4, **characterised in that** the active ingredient has an aqueous solubility at ambient temperature of less than 300 µg/ml, preferably less than 150 µg/ml, more preferably less than 125 µg/ml and, most preferably, less than 100 µg/ml.

6. Pharmaceutical composition according to claim 5, **characterised in that** the aqueous solubility is less than or equal to 20 µg/ml.

7. Pharmaceutical composition according to one of the preceding claims, **characterised in that** it contains no triglycerides and no phospholipids.

8. Pharmaceutical composition according to one of the preceding claims, **characterised in that** the fatty acid of the said or of at least one of the said metal salts is a carboxylic monoacid having from 8 to 18 carbon atoms.

9. Pharmaceutical composition according to claim 8, **characterised in that** the fatty acid is a saturated or unsaturated aliphatic acid and has from 16 to 18 carbon atoms, such as oleic acid.

10. Pharmaceutical composition according to one of the preceding claims, **characterised in that** the metal of the said or of at least one of the said metal salts is sodium or potassium.

11. Pharmaceutical composition according to claims 9 and 10, **characterised in that** the said or at least one of the said metal salts is sodium oleate.

12. Pharmaceutical composition according to one of the preceding claims, **characterised in that** the said or at least one of the said polyethylene glycol hydroxystearate(s) comprises 10 to 25 ethylene oxide units, preferably 15 ethylene oxide units.

13. Pharmaceutical composition according to one of the preceding claims, **characterised in that** it further comprises at least one polyoxyethylene compound as adjuvant(s), preferably a poloxamer or a sorbitan polyoxyethylene ester such as sorbitan polyoxyethylene monooleate with 20 ethylene oxide units (polysorbate 80).

14. Pharmaceutical composition according to one of the preceding claims, **characterised in that** it further comprises at least one solvent such as a polyethylene glycol, ethanol or a propylene glycol, as adjuvant(s).

15. Pharmaceutical composition according to claims 4 and 6, **characterised in that** the said active ingredient is a cyclic polypeptide such as cyclosporin A.

16. Pharmaceutical composition according to one of the preceding claims, **characterised in that** the ratio by mass (m/m) of the solubilising system to active ingredient is less than or equal to 15.

17. Pharmaceutical composition according to claim 16, **characterised in that** the ratio by mass (m/m) of solubilising system to active ingredient is less than or equal to 10, preferably less than or equal to 5.

18. Pharmaceutical composition according to one of the preceding claims, **characterised in that** the ratio by mass (m/m) of the said metal salt(s) to the said active ingredient is less than 1, preferably less than 0.05.

19. Pharmaceutical composition according to one of the preceding claims, **characterised in that** the ratio by mass (m/m) of the said polyethylene glycol hydroxystearate(s) to the said metal salt(s) is in a range from 10 to 130.

20. Process for preparing a pharmaceutical composition according to one of the preceding claims, **characterised in that** it comprises the addition of an active ingredient that is substantially insoluble in water, excluding Propofol, this active ingredient being dissolved beforehand in a solvent, to an aqueous mixture comprising:
(i) water, preferably in a fraction by mass in the said composition greater than or equal to 30%,
(ii) a solubilising system for said active ingredient which comprises at least one pharmaceutically acceptable monovalent metal salt of a fatty acid having 5 to 23 carbon atoms, such as sodium oleate, and at least one polyethylene glycol hydroxystearate, such as PEG-15 hydroxystearate, and
(iii) an adjuvant system comprising at least:
- one solvent which is identical to or different from the said solvent for dissolving the active ingredient, such as a polyethylene glycol, ethanol or propylene glycol, and/or
- a polyoxyethylene compound such as a poloxamer (e.g. poloxamer 188) and/or a sorbitan polyoxyethylene ester (e.g. sorbitan polyoxyethylene monooleate with 20 ethylene oxide units, also known as "polysorbate 80"), and/or
- a pH control agent, an osmolarity control agent and/or a structural agent.

## Patentansprüche

1. Pharmazeutische Zusammensetzung in Form einer wässrigen Emulsion, umfassend einen im Wesentlichen wasserunlöslichen Wirkstoff, ausgenommen Propofol, und ein Solubilisierungssystem für diesen Wirkstoff, wobei die Zusammensetzung zur intravenöser Injektion geeignet ist, **dadurch gekennzeichnet, dass** das Solubilisierungssystem wenigstens ein pharmazeutisch verträgliches monovalentes Metallsalz einer Fettsäure mit 5 bis 23 Kohlenstoffatomen und wenigstens ein Polyethylenglykolhydroxystearat umfasst.

2. Pharmazeutische Zusammensetzung nach Anspruch 1, **dadurch gekennzeichnet, dass** sie Wasser mit einem Massenanteil von gleich oder mehr als 30 %, bevorzugt von gleich oder mehr als 50 %, enthält, wobei die Zusammensetzung beispielsweise direkt in die Blutgefäße injiziert werden kann.

3. Pharmazeutische Zusammensetzung nach einem der vorherigen Ansprüche, **dadurch gekennzeichnet, dass** sie Mizellen mit einer mittleren Größe kleiner als 200 nm, bevorzugt kleiner als 100 nm, und besonders bevorzugt kleiner oder gleich 60 nm, umfasst.

4. Pharmazeutische Zusammensetzung nach einem der vorherigen Ansprüche, **dadurch gekennzeichnet, dass** der Wirkstoff auf einem bei Raumtemperatur festen Pulver basiert.

5. Pharmazeutische Zusammensetzung nach Anspruch 4, **dadurch gekennzeichnet, dass** der Wirkstoff bei Raumtemperatur eine Löslichkeit in Wasser hat, die kleiner als 300 µg/ml, bevorzugt kleiner als 150 µg/ml, besonders bevorzugt kleiner als 125 µg/ml, und ganz besonders bevorzugt kleiner als 100 µg/ml, ist.

6. Pharmazeutische Zusammensetzung nach Anspruch 5, **dadurch gekennzeichnet, dass** die Löslichkeit in Wasser kleiner oder gleich 20 µg/ml ist.

7. Pharmazeutische Zusammensetzung nach einem der vorherigen Ansprüche, **dadurch gekennzeichnet, dass** sie vollständig frei von Triglycerid(en) und Phospholipid(en) ist.

8. Pharmazeutische Zusammensetzung nach einem der vorherigen Ansprüche, **dadurch gekennzeichnet, dass** die Fettsäure des Metallsalzes oder wenigstens eines der Metallsalze eine Monocarbonsäure mit 8 bis 18 Kohlenstoffatomen ist.

9. Pharmazeutische Zusammensetzung nach Anspruch 8, **dadurch gekennzeichnet, dass** die Fettsäure eine gesättigte oder ungesättigte aliphatische Säure ist und 16 bis 18 Kohlenstoffatome aufweist, wie Oleinsäure.

10. Pharmazeutische Zusammensetzung nach einem der vorherigen Ansprüche, **dadurch gekennzeichnet, dass** das Metall des Metallsalzes oder wenigstens eines der Metallsalze Natrium oder Kalium ist.

11. Pharmazeutische Zusammensetzung nach den Ansprüchen 9 und 10, **dadurch gekennzeichnet, dass** das Metallsalz oder wenigstens eines der Metallsalze Natriumoleat ist.

12. Pharmazeutische Zusammensetzung nach einem der vorherigen Ansprüche, **dadurch gekennzeichnet, dass** das Polyethylenglykolhydroxystearat oder wenigstens eines der Polyethylenglykolhydroxystearate 10 bis 25 Ethylenoxideinheiten, bevorzugt 15 Ethylenoxideinheiten, aufweist.

13. Pharmazeutische Zusammensetzung nach einem der vorherigen Ansprüche, **dadurch gekennzeichnet, dass** sie weiterhin wenigstens eine Polyethylenverbindung als Adjuvans, vorzugsweise ein Poloxamer oder einen Polyoxyethylensorbitanester, wie Polyoxyethylensorbitanmonooleat mit 20 Oxyethyleneinheiten (Polysorbat 80), umfasst.

14. Pharmazeutische Zusammensetzung nach einem der vorherigen Ansprüche, **dadurch gekennzeichnet, dass** diese weiterhin wenigstens ein Lösungsmittel als Adjuvans, wie ein Polyethylenglykol, Ethanol oder ein Propylenglykol, umfasst.

15. Pharmazeutische Zusammensetzung nach einem der Ansprüche 4 und 6, **dadurch gekennzeichnet, dass** der Wirkstoff ein zyklisches Polypeptid, wie Cyclosporin A, ist.

16. Pharmazeutische Zusammensetzung nach einem der vorherigen Ansprüche, **dadurch gekennzeichnet, dass** das Massenverhältnis (m/m) von Solubilisierungssystem / Wirkstoff kleiner oder gleich 15 ist.

17. Pharmazeutische Zusammensetzung nach Anspruch 16, **dadurch gekennzeichnet, dass** das Massenverhältnis (m/m) von Solubilisierungssystem / Wirkstoff kleiner oder gleich 10, und bevorzugt kleiner oder gleich 5, ist.

18. Pharmazeutische Zusammensetzung nach einem der vorherigen Ansprüche, **dadurch gekennzeichnet, dass** das Massenverhältnis (m/m) des oder der Metallsalze zu Wirkstoff kleiner 1, und bevorzugt kleiner 0,05, ist.

19. Pharmazeutische Zusammensetzung nach einem der vorherigen Ansprüche, **dadurch gekennzeichnet, dass** das Massenverhältnis (m/m) des oder der Polyethylenglykolhydroxystearate zu dem oder den Metallsalzen in einem Bereich von 10 bis 130 liegt.

20. Verfahren zur Herstellung einer pharmazeutischen Zusammensetzung nach einem der vorherigen Ansprüche, **dadurch gekennzeichnet, dass** es die Zugabe eines im Wesentlichen wasserunlöslichen Wirkstoffs, ausgenommen Propofol, wobei der Wirkstoff zuvor in einem Lösungsmittel gelöst wurde, zu einer wässrigen Mischung umfasst, wobei die wässrige Mischung umfasst:
(i) Wasser, bevorzugt in einem Massenanteil in der Zusammensetzung von gleich oder mehr als 30 %,
(ii) ein Solubilisierungssystem für den Wirkstoff, umfassend wenigstens ein pharmazeutisch verträgliches monovalentes Metallsalz einer Fettsäure mit 5 bis 23 Kohlenstoffatomen, wie Natriumoleat, und wenigstens ein Polyethylenglykolhydroxystearat, wie PEG-15 Hydroxystearat, und
(iii) ein Adjuvanssystem, umfassend wenigstens:
- ein Lösungsmittel, das gleich oder verschieden ist zum Lösungsmittel für den Wirkstoff, wie Polyethylenglykol, Ethanol oder Propylenglykol, und/oder
- eine Polyoxyethylenverbindung, wie ein Poloxamer (z.B. Poloxamer 188), und/oder einen Polyoxyethylensorbitanester (z.B. Polyoxyethylensorbitanmonooleat mit 20 Oxyethyleneinheiten, auch "Polysorbat 80" genannt), und/oder
- ein pH-Wert-Kontrollmittel, ein Osmolaritätskontrollmittel und/oder ein Strukturmittel.
